Europäisches Patentamt

European Patent Office

Office européen des brevets

(11) Veröffentlichungsnummer: **0 303 791**

**A1**

(12) **EUROPÄISCHE PATENTANMELDUNG**

(21) Anmeldenummer: 88109597.0

(51) Int. Cl.⁴: **C12N 1/06**

(22) Anmeldetag: **16.06.88**

(30) Priorität: **21.08.87 CH 3220/87**

(43) Veröffentlichungstag der Anmeldung:
**22.02.89 Patentblatt 89/08**

(84) Benannte Vertragsstaaten:
**DE FR GB NL**

(71) Anmelder: **GEBRÜDER SULZER
AKTIENGESELLSCHAFT
Zürcherstrasse 9
CH-8401 Winterthur(CH)**

(72) Erfinder: **Rebsamen, Ernst Edwin
Dorfstrasse 18
CH-8800 Thalwil(CH)**
Erfinder: **Brändli, Ernst, Dr.
Brühlbergstrasse 99
CH-8400 Winterthur(CH)**

(74) Vertreter: **Dipl.-Ing. H. Marsch Dipl.-Ing. K.
Sparing Dipl.-Phys.Dr. W.H. Röhl
Patentanwälte
Rethelstrasse 123
D-4000 Düsseldorf 1(DE)**

(54) **Verfahren zum mechanischen Aufschluss von Zellen.**

(57) Die zu verarbeitende Suspension wird durch einen Arbeitsraum (6) geführt, welcher mit Mahlkörpern gefüllt ist und in einer Ringspaltkugelmühle vorgesehen ist. Der Zellaufschluss geschieht beim Rotieren eines walzenförmigen Rotors (3) konzentrisch zu einem walzenförmigen Stator (4). Diese begrenzen den Ringspalt (6). Nach der Behandlung der Suspension im Ringspalt wird das Material von den Mahlkugeln abgetrennt und abgeführt. Die Trennung wird an einem Trennelement vorgenommen, welches das aufgeschlossene Produkt durchlässt. Wichtig ist es, bei der Behandlung eine enge Verweilzeitverteilung, z.B. durch Erzeugen einer Taylor-Wirbelströmung, im Ringspalt zu erreichen und die Suspension in dieser Weise durch den Arbeitsraum zu führen. Die Betriebsparameter, wie die Geometrie des Ringspaltes (6), die Art der Mahlkörper und die Drehgeschwindigkeit des Rotors (3) werden dementsprechend gewählt.

FIG. 1

## VERFAHREN ZUM MECHANISCHEN AUFSCHLUSS VON ZELLEN

Die Erfindung bezieht sich auf ein Verfahren zum Aufschluss von Zellen und Zellverbänden.

Dabei kann es sich z.B. um Mikroorganismen unterschiedlichster Form handeln, wie z.B. um Bakterien mit kleinstem Durchmesser von 0.2 - 1 Mikron oder um Hefezellen mit kleinstem Durchmesser von 3 - 5 Mikron. Es ist bisher üblich den mechanischen Zellaufschluss in sogenannten Homogenisatoren auszuführen, wobei die Zellen zuerst einem Druck und nachher einer Entspannung ausgesetzt werden. Es ist auch bekannt, Zellen in Mühlen oder Destintegratoren verschiedener Bauart, z.B. in Kugel- bzw. Mahl-Hilfskörper-Mühlen, aufzuschliessen.

Diese Methoden befriedigen nicht vollkommen. Häufig ist die Verweilzeitverteilung der Zellen im Aufschluss-Apparat sehr inhomogen und/oder die Energiedissipationsdichte ist inhomogen im Arbeitsraum verteilt. Mechanische Methoden, bei denen man die Parameter nun so drastisch wählt, dass alle Zellen aufgeschlossen werden, führen häufig zu einer Schädigung des Produktes, z.B. durch einen thermischen Effekt, oder aber die Zellbruchstücke werden so klein, dass deren Abtrennung vom Produkt durch Filtration oder Zentrifugation vom enorm erschwert wird. Sind die Kräfte zu klein gewählt, wird die Behandlungszeit entsprechend lang, d.h. der Durchsatz sinkt, weil der Aufschlussprozess wiederholt durchgeführt werden muss.

Es ist Aufgabe der Erfindung, eine Methode zum mechanischen Zellaufschluss zu finden, bei der die einzelnen Zellen einen Arbeitsraum mit homogener Energiedissipationsdichte mit optimal kurzer Verweilzeit und enger Verweilzeitverteilung durchwandern und so möglichst gleichmässig aufgeschlossen werden.

Diese Aufgabe ist erfindungsgemäss durch Massnahmen erfüllt, die im kennzeichnenden Teil des Patentanspruches 1 angegeben sind.

Eine besonders homogene Behandlung, d.h. ein besonders gleichmässiger Zellaufschluss wird dadurch erzielt, dass die Strömungsverhältnisse im Arbeitsraum möglichst dem Pfropfströmungsverhalten nahekommen. Es wird erwartet, dass - bedingt durch die Ringspaltgeometrie und die Betriebsparameter -sich im Ringspaltraum der Ringspaltkugelmühle gemäss Anspruch 1 eine Taylor'sche Wirbelströmung ausbildet, mit entsprechender homogener Verweilzeitverteilung der suspendierten Zellen resp. Zellverbände .

Im weiteren wird der Erfindungsgegenstand näher beschrieben und erklärt. Die Beschreibung bezieht sich auf die beiliegende Zeichnung, welche zeigt:

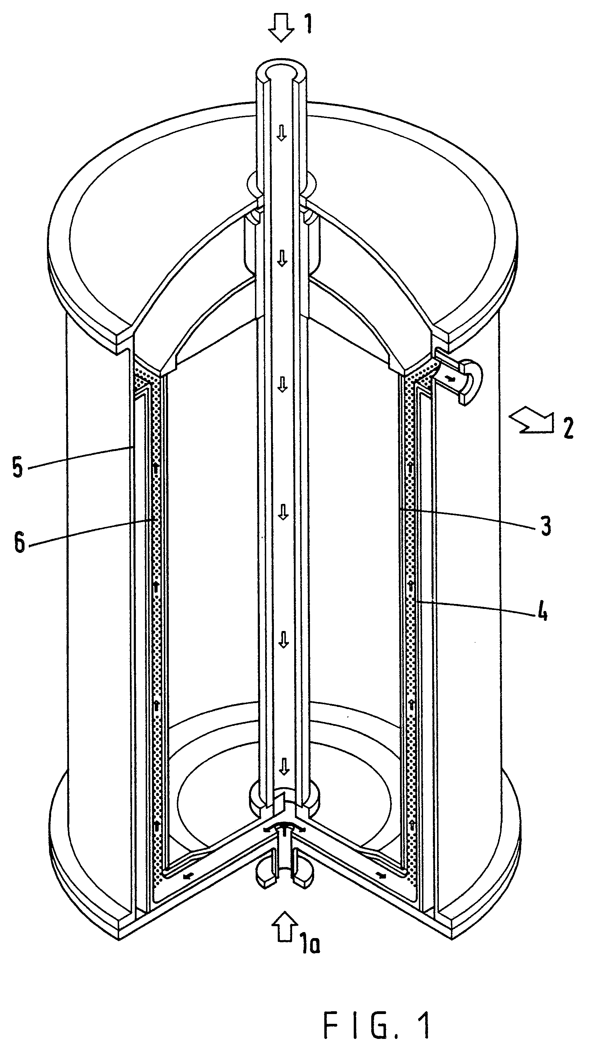

Figur 1 eine geeignete Ringspaltkugelmühle, schematisch dargestellt in teilweiser Schnittzeichnung.

Die zu behandelnde Zellsuspension wird durch einen Einspeisungsweg (1) in den Arbeitsraum, d.h. den Ringspalt (6), wie in der Zeichnung mit Pfeilen angedeutet, geleitet und durch diesen geführt, in diesem behandelt und via einen Ueberlauf (2) abgeführt. Der Zellaufschluss erfolgt im Ringspalt (6) bei drehendem Rotor (3) relativ zum Stator (4), die ihrerseits den Ringspalt (6) begrenzen.

Vor dem Ueberlauf (2) ist ein nicht eingezeichnetes Trennelement vorgesehen, z.B. ein Sieb, welches die Suspension mit den aufgeschlossenen Zellen durchlässt, jedoch die Mahlkörper zurückhält.

Es ist in der Zeichnung nicht dargestellt, aber leicht vorstellbar, dass vor diesem Trennelement ein Weg anfängt, durch welchen die dort abgetrennten Mahlkörper zurück zum Ringspalt (6) geführt werden. Es ist weiterhin von Vorteil, wenn die Kugeln unterwegs gewaschen, gekühlt, kontrolliert, gegebenenfalls nicht mehr geeignete aussortiert werden. Die so behandelten Mahlkörper werden im vorliegenden Fall der zuströmenden Suspension bei (1a) zugeführt, jedenfalls aber vor dem Eintritt der Suspension in den Ringspalt.

Zum Fördern der Suspension durch den Ringspalt ist eine nicht gezeichnete Pumpe vorgesehen.

Wie aus der Zeichnung zu ersehen ist, wird der Stator (4) von einem Kühlmantel (5) umgeben. Dieser wird zum Thermostatieren verwendet, indem z.B. ein Kühlmedium in geeigneter Weise durch diesen Raum gepumpt wird, um die Suspension im Ringspalt bei der gewünschten bzw. notwendigen Temperatur, bzw. innerhalb der für das entsprechende Produkt zulässigen Grenzen zu halten. Meist ist Kühlung notwendig, da bei der Behandlung der Zellen Reibungswärme entsteht.

Es ist wünschenswert und oft auch notwendig, den Zellaufschluss unter sterilen Bedingungen durchzuführen. Dies muss in einem sterilisierten Arbeitsraum geschehen, indem in der Ringspaltkugelmühle alle mit dem zu verarbeitenden Material in Berührung kommenden Flächen mit Dampf sterilisiert werden. Dazu weist der Apparat entsprechende Dichtungen, Dampfanschlüsse sowie Ventile und Luftfilter auf, wie es bei dieser Sterilisationstechnik üblich ist und deshalb in der Zeichnung nicht gezeigt wird.

Um die sterilen Bedingungen sicher einhalten zu können und auch erhöhten Sicherheitsanforderungen zu genügen, kann der Arbeitsraum der Ringspaltkugelmühle gegen die Umgebung abgekapselt werden. Bei dieser Ausführung kann der Rotor mittels eines rotierenden magnetischen Feldes zum Rotieren gebracht werden. Auch diese Massnahme, an sich wohlbekannt, ist in der Zeichnung einfachheitshalber nicht gezeigt.

Beispiel:

In einer Ringspaltkugelmühle RSK 10 - 25 wurde Bäckerhefe aufgeschlossen, mit dem Ziel eines quantitativen Zellaufschlusses in einem Durchgang.

Der Rotor wies einen Durchmesser von 10 cm und eine Länge von 25 cm auf und wurde bei verschiedenen Drehzahlen betrieben. Rotor und Stator waren aus rostfreiem Stahl angefertigt. Der Ringspalt hatte eine lichte Weite von 0.6 cm.

Die Mahlkugeln bestanden aus Glas und wiesen Durchmesser von 0.5 - 0.75 mm auf. Ihre Dichte betrug 2.5 g/cm³ und als Füllgrad wählte man 70 % bezogen auf das totale Volumen des Arbeitsraumes.

Die Suspension enthielt 40 % v/v Bäckerhefe und wurde bei 5°C mittels einer Peristaltikpumpe der Mühle kontinuierlich zugeführt. Die Effizienz des Zellaufschlusses wurde überprüft durch Messung der in Lösung gehenden Zellproteine und verglichen mit einem vollständigen Zellaufschluss im Batchansatz.

Bei einer Drehzahl von 3500 rpm, was einer Umfangsgeschwindigkeit von rund 18.5 m/s entspricht, wurde bei einem Durchsatz von 20 l/h, entsprechend einer theoretischen Verweilzeit von 1.5 Minuten bezogen auf das Nutzvolumen (Nutzvolumen = Totalvolumen - Kugelvolumen), ein Aufschlussgrad > 95 % erzielt. Der Temperaturanstieg in der Suspension konnte bei Verwendung von Kühlsole von - 10°C bei < 5°C gehalten werden.

Der hohe Aufschlussgrad bei sehr kurzer Verweilzeit und die effiziente Wärmeabführung werden auf die speziellen Strömungsverhältnisse (Taylor-Wirbelströmung) im Ringspalt zurückgeführt.

**Ansprüche**

1. Verfahren zum mechanischen Aufschluss von Zellen und Zellverbänden, gekennzeichnet durch Führen der zu verarbeitenden Suspension durch einen mit Mahlkörpern gefüllten Arbeitsraum (6) einer Ringspaltkugelmühle mit rotierendem walzenförmigem Rotor (3), konzentrisch zu einem walzenförmigen Stator (4) einen ringförmigen Spalt begrenzend, wobei die im Ringspalt (6) behandelte Suspension mit den aufgeschlossenen Zellen und Zellverbänden nach erfolgtem Aufschluss im Ringspalt von den Mahlkörpern abgetrennt und abgeführt wird.

2. Verfahren nach Anspruch 1, dadurch gekennzeichnet, dass die Abtrennung der Mahlkörper aus dem Produktstrom mittels eines im Wege des abzuführenden Produktstromes vorgesehenen Trennelementes ausgeführt ist.

3. Verfahren nach Anspruch 1, dadurch gekennzeichnet, dass die abgetrennten Mahlkörper in einem geschlossenen Kreislauf zurück zum Ringspalt (6) geführt werden, wobei sie unterwegs behandelt werden.

4. Verfahren nach Anspruch 1, dadurch gekennzeichnet, dass bei der Behandlung der Suspension im Ringspalt (6) eine Taylor'sche Wirbelströmung aufrechterhalten wird, wozu die Betriebsparameter wie die Geometrie des Ringspaltes (6), die Art der Mahlkörper und die Drehgeschwindigkeit des Rotors (3) entsprechend gewählt werden.

5. Verfahren nach Anspruch 1, dadurch gekennzeichnet, dass dabei die Temperatur der zu behandelnden Suspension, bzw. der zu behandelnden Zellen, innerhalb eines für das jeweilige Produkt zulässigen Temperaturbereiches gehalten wird.

6. Verfahren nach Anspruch 1, dadurch gekennzeichnet, dass dies in einem sterilen Apparat geschieht, indem alle produktberührten Teile des Arbeitsraumes mit Dampf sterilisiert werden können, wozu entsprechende Dichtungen, Anschlüsse und Filter am Apparat vorgesehen sind.

7. Verfahren nach Anspruch 1, dadurch gekennzeichnet, dass dies in einem gegen die Umgebung vollkommen abgeschlossenen Arbeitsraum vor sich geht, wobei der Rotor (3) mittels eines rotierenden magnetischen Feldes oder einer analogen Vorrichtung zur Kraftübertragung in Rotation gebracht wird.

8. Verfahren nach Anspruch 1, gekennzeichnet durch Anwendung einer Ringspaltkugelmühle, die im wesentlichen aus der DE-OS 28 48 479 bekannt ist.

FIG. 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | FR-A-2 409 089 (MICROPROCESS AG) <br> * Ansprüche; Figuren in der Anmeldung erwähnt * <br> --- | 1-8 | C 12 N 1/06 |
| A | CH-A- 611 533 (INSTITUT BIOKHIMII I FIZIOLOGII MIKROORGANIZMOV AKADEMII NAUK SSSR) <br> * Ansprüche; Figuren; Zeile 2, Spalte 1, Zeilen 39-47 * <br> --- | 1-8 | |
| A | DE-A-2 102 481 (G. FRIEDRICH) <br> --- | | |
| A | FR-A-1 576 299 (VYZKUMNY USTAV ORGANICKYCH SYNTEZ PARDUBICE RYBITVI) <br> --- | | |
| A | US-A-3 190 568 (D. FREEDMAN et al.) <br> ------ | | |

RECHERCHIERTE SACHGEBIETE (Int. Cl.4)

C 12 N
C 12 M
B 02 C

Der vorliegende Recherchenbericht wurde für alle Patentansprüche erstellt

| Recherchenort | Abschlußdatum der Recherche | Prüfer |
|---|---|---|
| DEN HAAG | 18-08-1988 | COUCKE A.O.M. |